# EUROPÄISCHE PATENTSCHRIFT

(11) **EP 2 228 087 B1**
(45) Veröffentlichungstag und Bekanntmachung des Hinweises auf die Patenterteilung: **27.02.2013**
(21) Anmeldenummer: 10155287.5
(22) Anmeldetag: 03.03.2010
(51) Int. Cl.: A61M 16/06

(54) **Beatmungsmaske**
Breathing mask
Masque respiratoire

(30) Priorität: 05.11.2009 DE 202009015062 U; 09.03.2009 DE 202009003112 U
(43) Veröffentlichungstag der Anmeldung: 15.09.2010
(73) Patentinhaber: Airtec Beatmungshilfen GmbH & Co KG, 45468 Mülheim an der Ruhr (DE)
(72) Erfinder: Menzel, Stefan, Dipl.-Ing., 45239 Essen (DE); Jansen, Ludger, 45239 Essen (DE)
(74) Vertreter: Cohausz & Florack

(56) Entgegenhaltungen:
- EP-A2- 1 147 782
- WO-A2-2006/113321
- DE-A1-102006 011 630
- US-A- 5 243 971
- US-A1- 2002 053 347
- US-A1- 2006 130 844
- US-A1- 2008 060 653

## Beschreibung

Die Erfindung betrifft eine Beatmungsmaske für die Mund-Nasen- oder Nasenbeatmung, mit einem ein Anschlussstück für einen Beatmungsschlauch aufweisenden Maskenkörper aus einem formsteifen Material und einem mit dem Maskenkörper gesichtsseitig verbundenen, an das Gesicht des Patienten anlegbaren Maskeneinsatz aus einem flexiblen Material, wobei der Maskenkörper ein Verwirbelungsvolumen aufweist, wobei der Maskeneinsatz eine umlaufende Dichtlippe zur abdichtenden Anlage des Maskeneinsatzes am Gesicht des Patienten aufweist und wobei der Maskeneinsatz an seiner gesichtsabgewandten Seite mit dem Maskenkörper über einen am Maskeneinsatz vorgesehenen zumindest teilweise umlaufenden Flansch und einen korrespondierenden, an dem Maskenkörper vorgesehenen Flansch dicht verbunden ist wobei der Maskenkörper als vorkonfektionierter Maskenkörper ausgebildet ist und der Maskeneinsatz individuell an die Gesichtsanatomie des Patienten angepasst ausgebildet ist, wobei, der Maskenkörper ein Verwirbelungsvolume aufweist, wobei die über einen Beatmungsschlauch zugeführte Beatmungsluft zunächst verwirbelt wird, so dass sich die Beatmungsluft erwärmt, bevor die Beatmungsluft über Öffnungen des Maskeneinsatzes in die Nase des Patienten einströmt.

Mund-Nasen- oder Nasenbeatmungsmasken der vorstehend beschriebenen Art sind in der Medizintechnik seit Jahren bekannt. Neben dem Einsatz in der Intensivmedizin kommen sie insbesondere bei der Klinik- und Heimbehandlung chronischer respiratorischer Erkrankungen zum Einsatz, bei denen der Patient beispielsweise nachts auf eine Beatmungshilfe angewiesen ist. Im Vordergrund steht hierbei die Behandlung der Schlafapnoe und der Heimbeatmung, z.B. bei COPD-Patienten. Beatmungsmasken der eingangs genannten Art werden aber auch bei COPD-Patienten oder auch MS-Patienten eingesetzt.

Eine aus der Praxis bekannte, individuell angepasste Mund-Nasen-Beatmungsmaske umfasst einen Silikonvollkörper als flexibles Maskenelement, welcher derart geformt ist, dass er über seine Innenseite unmittelbar an Mund und Nase des Patienten anliegt. Bei dieser Maske ist darüber hinaus an der Innenseite des Silikonvollkörpers ein Mundstück angeformt, das beim Tragen der Maske in den Mund des Patienten eingeführt wird. Ferner umfasst die vorstehend beschriebene Maske ein oberhalb der Nase und unterhalb des Mundes angeordnetes Kunststoffversteifungselement, welches außenseitig in den Silikonvollkörper der Beatmungsmaske eingelassen ist und der Befestigung von Haltebändern dient.

Solche Masken weisen hinsichtlich des Tragekomforts einige gravierende Nachteile auf. So durchströmt die Beatmungsluft den Maskenvollkörper aufgrund der direkten Anlage an Mund und Nase des Patienten mit vergleichsweise hoher Geschwindigkeit, was bei der Nasenatmung langfristig zu einer Schleimhautaustrocknung führt. Aufgrund der massiven Ausführung des Maskenkörpers kommt es ferner immer wieder zu Druckstellen an Nasenwurzel und Nasenrücken. Zudem führt die unzureichende Flexibilität des Maskenkörpers bei einseitiger Belastung, wie sie beim Schlafen des Patienten auf der Seite vorkommt, zu Undichtigkeiten, denen einerseits nur durch eine Beatmungsdruckerhöhung oder durch ein Anziehen der Haltebänder, was seinerseits wieder zu stärker ausgebildeten Druckstellen führt, begegnet werden kann. Schließlich kann es durch die Auflage der Maske im Bereich der Frontzähne zu dauerhaften Schäden und/oder Druckschmerzen an den Zahnhälsen kommen. Ein weiteres Problem ist die Versorgung von klaustrophobischen Patienten, die zu eng anliegende Masken nicht tolerieren.

Eine individuell angepasste Mund-Nase-Beatmungsmaske ist aus der DE 20 2006 016 737 U1 bekannt. Diese umfasst ein flexibles, an die Gesichtsanatomie des Patienten individuell angepasstes Maskenelement und ein außen auf dem Maskenelement flächig aufliegendes Versteifungselement, wobei das Maskenelement als ein Vollkörper aus einem elastischen Material ausgebildet ist und eine den Mund-Nasenbereich umschließende, umlaufende innere Dichtlippe umfasst. Diese Beatmungsmaske zeichnet sich durch einen hohen Tragekomfort auch über längere Zeiträume aus und bewirkt einen Druckstellenfreien Sitz am Gesicht des Patienten bei guter Abdichtung. Allerdings muss sie in einem vergleichsweise aufwändigen Herstellungsverfahren produziert werden, so dass sie vergleichsweise teuer in der Anschaffung ist.

Dokumente EP 1 147 782 A2 und US 5 243 971 A zeigen Beatmungsmasken relevant für den Stand der Technik.

Hiervon ausgehend liegt der Erfindung die Aufgabe zugrunde, eine Beatmungsmaske für die Mund-Nasen- oder Nasenbeatmung anzugeben, die einen hohen Tragekomfort bei gleichzeitig hoher Dichtigkeit gewährleistet und günstig in der Anschaffung ist.

Die Aufgabe wird mit einer Beatmungsmaske nach dem Oberbegriff des Patentanspruches 1 dadurch gelöst, dass der Maskenkörper als vorkonfektionierter Maskenkörper ausgebildet ist und der Maskeneinsatz individuell an die Gesichtsanatomie des Patienten angepasst ausgebildet ist.

Der besondere Vorteil der erfindungsgemäßen Beatmungsmaske ist, dass sie erfindungsgemäß zu einem erheblichen Teil auf konfektionierte Komponenten, nämlich auf den aus einem formsteifen Material gefertigten Maskenkörper, zurückgreift.

Ein solcher ist bei kommerziellen Beatmungsmasken, wie sie beispielsweise in Schlafkliniken verwendet werden, üblich und ist in der Regel in mehreren Standardgrößen verfügbar. Hierdurch lassen sich die Herstellungskosten wirksam reduzieren. Vorkonfektionierte Maskenkörper werden beispielsweise von der ResMed GmbH & Co. KG, Martinsried angeboten.

Geeignete Materialien für den formsteifen Maskenkörper sind verschiedene Kunststoffe, wie beispielsweise Polycarbonat. Durch die individuell an die Gesichtsanatomie des Patienten angepasste Form des Maskeneinsatzes wird ein hervorragender Tragekomfort infolge der optimalen Passform des Maskeneinsatzes bei gleichzeitiger absolut dichter Anlage der Dichtlippe am Gesicht des Patienten erzielt.

Es gibt mehrere Möglichkeiten, den erfindungsgemäß individuell an die Gesichtsanatomie des Patienten angepasst ausgebildeten Maskeneinsatz auszugestalten. Nach einer ersten Ausgestaltung ist der Maskeneinsatz ringförmig mit einer zentralen Aussparung zum Einströmen der Beatmungsluft ausgebildet. Dies bedeutet, dass der Maskeneinsatz maßgeblich durch eine umlaufende, individuell an die Gesichtsanatomie des Patienten angepasste Dichtlippe, die auf der gesichtsabgewandten Seite einen zumindest teilweise umlaufenden Flansch zur gasdichten Befestigung am Maskenkörper umfasst, gebildet wird. Die umlaufende Dichtlippe kann bezogen auf den Maskeneinsatz nach innen verbreitert sein, um zusätzliche Auflagefläche für einen komfortablen Sitz des Maskeneinsatzes am Gesicht des Patienten zu schaffen. Durch eine solche Ausgestaltung des individuell an die Gesichtsanatomie des Patienten angepassten Maskeneinsatzes wird bei minimalem Materialaufwand ein optimaler Sitz am Gesicht des Patienten sichergestellt, so dass einerseits eine optimal dichte Anlage des Maskeneinsatzes am Gesicht des Patienten und andererseits ein hoher Tragekomfort mit vergleichsweise geringer Berührungsfläche zwischen Maskeneinsatz und Gesichtshaut sowie eine sichere Beatmung des Patienten gewährleistet ist.

Der Maskeneinsatz ist als Vollkörper ausgebildet, wobei der von der umlaufenden Dichtlippe umschlossene Bereich individuell an die Gesichtsanatomie des Patienten angepasst ausgebildet ist. Demnach ist der Maskeneinsatz flächig ausgebildet, wobei es sich versteht, dass in dem als Vollkörper ausgebildeten Maskenkörper eine oder mehrere Öffnungen zum Einströmen von Beatmungsluft vorgesehen sind. Die mit einem als Vollkörper ausgebildeten Maskeneinsatz versehene Beatmungsmaske kann beispielsweise für die Mund-Nasen-Beatmung ausgelegt sein. Dementsprechend umschließt die umlaufende Dichtlippe des Maskeneinsatzes bevorzugt einen individuell an die Anatomie des Mund-Nasenbereichs des Patienten angepassten Bereich. Im Falle einer Beatmungsmaske für die reine Nasenbeatmung versteht es sich, dass die umlaufende Dichtlippe lediglich einen individuell an die Anatomie des Nasenbereichs des Patienten angepassten Bereich des Maskenkörpers umschließt.

Bevorzugt ist auch die Dichtlippe individuell an die Gesichtsanatomie des Patienten angepasst ausgebildet. Hierdurch werden eine druckstellenfreie, vollständig abdichtende Auflage auf dem Gesicht des Patienten und ein besonders hoher Tragekomfort gewährleistet.

Nach einer besonders vorteilhaften Ausgestaltung der Erfindung umfasst der von der umlaufenden Dichtlippe umschlossene Bereich des als Vollkörper ausgebildeten Maskeneinsatzes zumindest eine Abformung der Nasenpartie des Patienten, wobei zwei Öffnungen zum unmittelbaren Einströmen der Atmungsluft in die Nasenlöcher vorgesehen sind. Als Vollkörper ausgebildete Maskeneinsätze mit einer derart gewählten Form zeichnen sich durch einen besonders hohen Tragekomfort und eine infolge des an die Nasenanatomie des Patienten individuell angepassten inneren Bereichs besonders gute Passform bei gleichzeitig kompakten Außenabmessungen aus. Es versteht sich dabei, dass sich derartige Maskeneinsätze sich insbesondere für die reine Nasenbeatmung eignen.

Nach einer weitergehenden Ausgestaltung sind die zwei Öffnungen von stutzenartigen Verlängerungen umgeben, die in die Nasenlöcher des Patienten einführbar sind. Eine solche Ausformung wirkt sich nicht negativ auf den Tragekomfort aus und sorgt darüber hinaus für eine besonders definierte Anlage des Maskeneinsatzes an der Nase des Patienten.

Der Maskenkörper weist ein Verwirbelungsvolumen auf., Dementsprechend strömt die in die Maske einströmende Luft nicht unmittelbar mit hoher Geschwindigkeit in die Nase ein, sondern kann sich in den Verwirbelungsvolumen zunächst erwärmen, wodurch die Beatmung angenehmer gestaltet ist und ein Austrocknen der Nasenschleimhäute wirksam verhindert wird.

Nach einer weiteren Ausgestaltung der Erfindung umfasst die Beatmungsmaske einen Stützring zur Sicherung des Flansches des Maskeneinsatzes an dem korrespondierenden Flansch des Maskenkörpers. Hierdurch wird eine einfache und sichere Befestigung des Maskeneinsatzes am Maskenkörper sichergestellt. Der Stützring kann aus dem gleichen Material wie der Maskenkörper gefertigt sein. Bevorzugt kann zur für eine sichere und lösbare Verbindung zwischen Maskenkörper und Maskeneinsatz zwischen dem Stützring und dem Maskenkörper eine Schnappverbindung hergestellt werden. Eine dichte Verbindung zwischen Maskeneinsatz und Maskenkörper ist jedoch auch ohne Stützring möglich, was den Herstellungs- und Teileaufwand verringert.

Nach einer weiteren Ausgestaltung der Erfindung weist der am Maskeneinsatz vorgesehene zumindest teilweise umlaufende Flansch eine Nut auf, in die eine an dem korrespondierenden Flansch des Maskenkörpers vorgesehene Feder eingreift. Die Nut ist dabei bevorzugt entlang des zumindest teilweise umlaufenden Flansches ausgerichtet. Durch eine derart bewerkstelligte Nut-Feder-Verbindung ist ausgeschlossen, dass das Maskenelement bei unsachgemäßer Handhabung versehentlich in das Verwirbelungsvolumen des Maskenkörpers eingedrückt wird, wodurch es zu Undichtigkeiten kommen kann.

Nach einer weiteren Ausgestaltung der Erfindung weist der Maskenkörper eine Stirnauflage auf. Durch eine solche Stirnauflage wird einerseits der Tragekomfort der Beatmungsmaske erhöht. Andererseits wird die Position der Beatmungsmaske hinsichtlich einer Verschwenkbarkeit um eine in der Frontalebene des Patienten liegende Achse, die einem nicht abdichtenden Sitz der Beatmungsmaske führen kann, stabilisiert. Zweckmäßigerweise ist dabei der Abstand der Stirnauflage von dem Maskeneinsatz auf Basis gesichtsanatomischer Vorgaben gewählt.

Nach einer weitergehenden Ausgestaltung ist die Stirnauflage arretierbar gelenkig mit dem übrigen Maskenkörper verbunden. Hierdurch können unterschiedliche Gesichtsanatomien berücksichtigt (fliehende Stirn, hervortretende Stirn usw.) werden.

Um den Tragekomfort der Stirnauflage zu verbessern, kann ferner vorgesehen sein, dass die Stirnauflage wenigstens ein Stirnpolster aus einem flexiblen Material aufweist. Hierbei kann es sich um das gleiche Material wie im Falle des flexiblen Maskenkörpers handeln. Alternativ kann das wenigstens eine Stirnpolster als Gelkissen ausgebildet sein.

Für den Maskenkörper - und für ein ggf. vorgesehenes Stirnpolster - wird bevorzugt ein biokompatibles Material, insbesondere ein biokompatibles medical Grade 2-Silikon-Elastomer, verwendet. Hierdurch können unerwünschte, ggf. allergische, Hautreaktionen minimiert werden.

Im Folgenden wird die Erfindung anhand einer ein Ausführungsbeispiel darstellenden Zeichnung näher erläutert.
Es zeigen:
- Fig. 1: eine Nasenbeatmungsmaske mit einem individuell an die Gesichtsanatomie des Patienten angepassten Maskeneinsatz in Frontansicht
- Fig. 2: die Nasenbeatmungsmaske aus Fig. 1 in Seitenansicht,
- Fig. 3: den Maskeneinsatz der Nasenbeatmungsmaske aus Fig. 1 in Frontansicht,
- Fig. 4: den Maskeneinsatz aus Fig. 3 in rückwärtiger Ansicht,
- Fig. 5: den Maskeneinsatz aus Fig. 3 in seitlicher Ansicht,
- Fig. 6: den Maskeneinsatz aus Fig. 3 im Sagittalschnitt,
- Fig. 7: einen Maskeneinsatz für eine Nasenbeatmungsmaske in einer von Fig. 3 abweichenden Ausgestaltung in Frontansicht und
- Fig. 8: den Maskeneinsatz aus Fig. 7 im Sagittalschnitt.

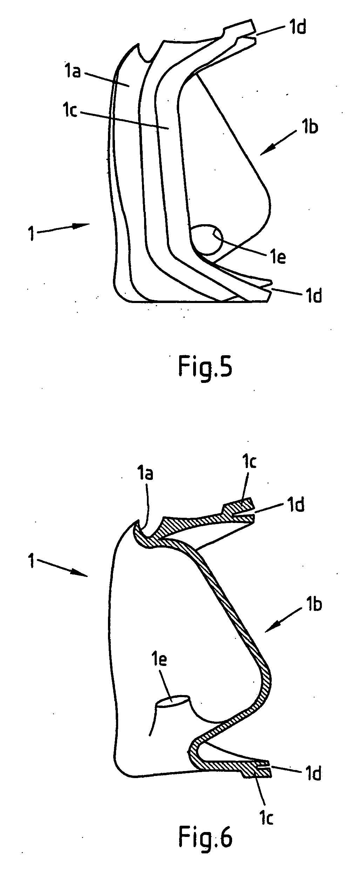

In Figur 5 ist eine Nasenbeatmungsmaske mit einem individuell an die Gesichtsanatomie des Patienten angepassten Maskeneinsatz 1 in Frontansicht dargestellt. Die Beatmungsmaske umfasst ferner einen Maskenkörper 2, welcher seinerseits ein Anschlussstück 2a für einen Beatmungsschlauch (nicht dargestellt) aufweist und aus einem formsteifen Material, insbesondere einem Kunststoff, speziell Polycarbonat, gefertigt ist. Während der Maskeneinsatz 1, wie erwähnt, individuell an die Gesichtsanatomie des Patienten angepasst ist, ist der Maskenkörper 2 vorkonfektioniert ausgebildet und in Standardgrößen erhältlich. Vorliegend handelt es sich bei dem vorkonfektionierten Maskenkörper 2 um den Maskenkörper "Ultra Mirage™ II Nasal Mask" der ResMed GmbH & Co. KG, Martinsried. Es versteht sich, dass im Rahmen der vorliegenden Erfindung auch auf vorkonfektionierte Maskenkörper anderer Fabrikate zurückgegriffen werden kann. Entscheidend ist die Kombination aus einem vorkonfektionierten Maskenkörper 2 und einem individuell an die Gesichtsanatomie des Patienten angepasst ausgebildeten Maskeneinsatz 1.

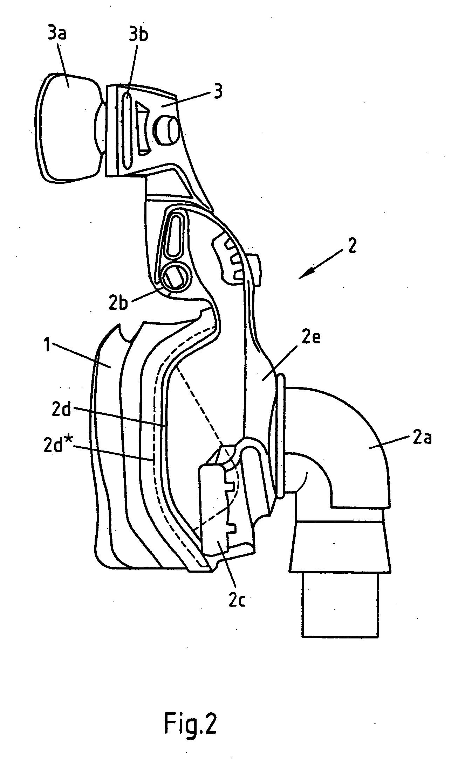

Direkt am Anschlussstück 2a des Maskenkörper 2 wird durch die ausgewölbte Form des Maskenkörpers ein Verwirbelungsvolumen 2e definiert (s. Fig. 2), in welchem die über den Beatmungsschlauch zugeführte Beatmungsluft zunächst erwärmt wird, bevor sie in die Nase des Patienten einströmt.

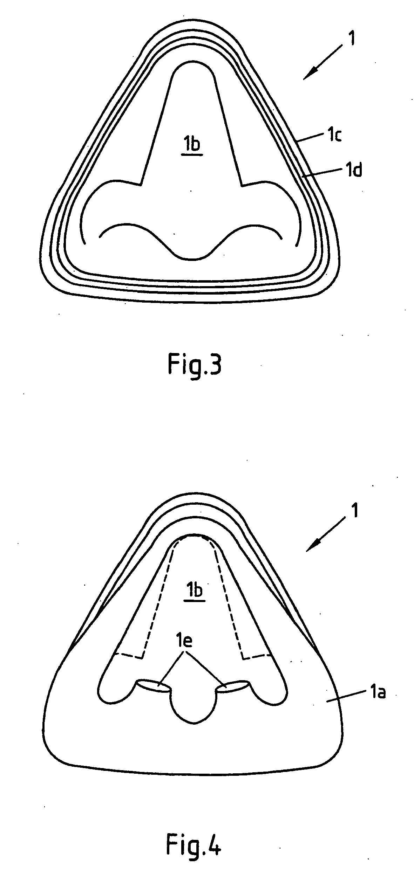

Ferner umfasst die Beatmungsmaske einen flexiblen Maskeneinsatz 1 mit einer umlaufenden Dichtlippe 1a und einem inneren Bereich 1b, welche beide individuell an die Gesichtsanatomie des Patienten angepasst sind, wie im Zusammenhang mit den Fig. 3 bis 6 noch näher erläutert wird.

Die Beatmungsmaske weist zudem eine arretierbar gelenkig mit dem Maskenkörper 2 verbundene Stirnauflage 3 auf, mittels derer weitere Abstützpunkte der Beatmungsmaske am Gesicht des Patienten, vorliegend dem Stirnbereich, definiert werden. Für einen komfortablen Sitz der Beatmungsmaske weist die Stirnauflage 3 zwei Stirnpolster 3a auf, welche aus einem flexiblen Material gefertigt sein können, beispielsweise aus dem Material, aus dem auch der Maskeneinsatz 1 gefertigt ist, bevorzugt also wiederum einem biokompatiblen medical Grade 2-Silikon-Elastomer.

Wie bereits erwähnt, ist die Stirnauflage 3 arretierbar gelenkig mit dem Maskenkörper 2 verbunden. Hierdurch können unterschiedliche Gesichtsanatomien berücksichtigt werden, beispielsweise eine fliehende Stirn, bei der die Stirnauflage aus der Flucht mit dem Maskenkörper 2 in Richtung des Gesichtes des Patienten verschwenkt werden muss, oder eine stark hervortretende Stirn, in welcher die Stirnauflage mit dem Maskenkörper 2 im Wesentlichen fluchtet. Das Gelenk zur Verschwenkung der Stirnauflage 3 ist mit dem Bezugszeichen 2b gekennzeichnet.

Zur komfortablen Befestigung der Beatmungsmaske am Kopf des Patienten werden, wie aus dem Stand der Technik an sich bekannt, flexibel ausgebildete Trageriemen 4 verwendet, welche vorliegend über Klickschnallen 4a an dem Maskenkörper 2 befestigt sind. Hierfür vorgesehene Steckaufnehmer 2c sind an den Maskenkörper 2 im Bereich des Beatmungsschlauchanschlusses 2a vorgesehen. Zur Erhöhung des Tragekomforts und gleichzeitig zur Erhöhung eines abdichtenden Sitzes der Beatmungsmaske am Kopf des Patienten sind auch im Bereich der Stirnauflage 3 schlitzartige Riemendurchführungen 3b vorgesehen.

Zur sicheren und abdichtenden Befestigung des Maskeneinsatzes 1 weist der Maskenkörper 2 gesichtsseitig einen Flansch 2d mit umlaufender Feder 2d* vor, welche in eine in einem umlaufenden Flansch 1c des Maskeneinsatzes 1 vorgesehene Nut 1d eingesteckt werden kann, wie im Zusammenhang mit den Fig. 3 noch im Detail erläutert wird. Zur Sicherung des flexiblen Maskeneinsatzes 1 an dem Maskenkörper 2 kann zusätzlich vorgesehen sein, dass ein Stützring (nicht dargestellt) über den Flansch 1c des Maskeneinsatzes 1 geschoben wird und beispielsweise mittels einer Schnappverbindung am Maskenkörper 2 befestigt wird. Eine sichere und dichte Befestigung des Maskeneinsatzes 1 am Maskenkörper 2 kann - wie vorliegend - jedoch auch ohne einen derartigen Stützring erzielt werden.

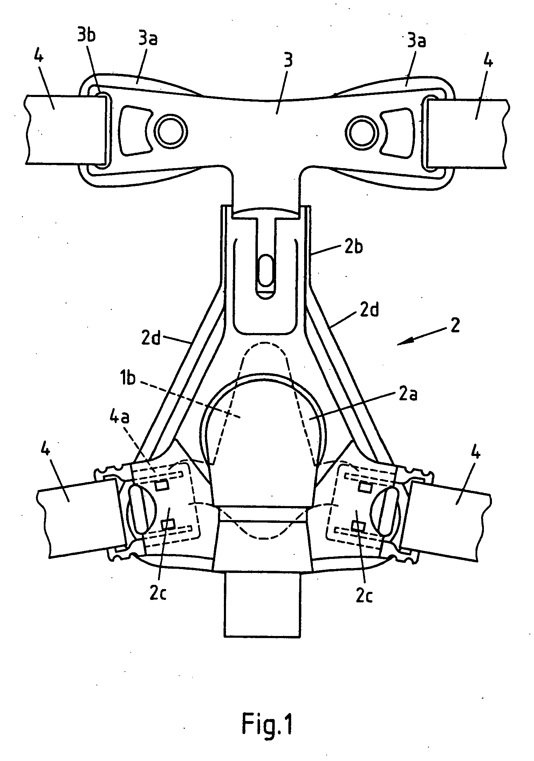

In Figur 2 ist die Beatmungsmaske aus Figur 1 in einer Seitenansicht dargestellt. Gut erkennbar ist hier das Gelenk 2b, über welches Stirnauflage 3 und Maskenkörper 2 zueinander verschwenkt werden können.

In Figur 3 ist nun der Maskeneinsatz 1 für die Nasenbeatmungsmaske in Frontansicht dargestellt. Der Maskeneinsatz 1 ist aus einem flexiblen Material, bevorzugt wiederum biokompatiblem medical Grade 2-Silikon-Elastomer, gefertigt und ferner als Vollkörper ausgebildet. Er umfasst eine umlaufende Dichtlippe 1a (vergleiche Figur 4) sowie einen von der umlaufenden Dichtlippe 1a umschlossenen Bereich 1b. Vorliegend sind sowohl der umschlossene Bereich 1b als auch die umlaufende Dichtlippe 1a individuell an die Gesichtsanatomie des Patienten angepasst ausgebildet, wodurch optimale Dichtigkeit bei gleichzeitig sehr hohem Tragekomfort gewährleistet wird.

Wie in Figur 1 erkennbar, bildet der Maskeneinsatz 1 die individuelle Nasenpartie des Patienten ab. Hierdurch wird ein noch weiter verbesserter Tragekomfort der Beatmungsmaske bei absolut dichter Anlage der Dichtlippe 1a am Gesicht des Patienten erzielt.

Wie sich der Figur 3 ferner entnehmen lässt, umfasst der Maskeneinsatz 1 auf seiner in Figur 3 dargestellten Vorderseite einen umlaufenden Flansch 1c, in den seinerseits, wie bereits erwähnt, eine umlaufende Nut 1d eingeformt ist. Mittels des Flansches 1c und der darin eingeformten Nut 1d kann der Maskeneinsatz 1 an dem am Maskenkörper 2 vorgesehen korrespondierenden Flansch 2d mit Feder 2d* dicht und sicher befestigt werden (vgl. Fig. 2).

Wie bereits erwähnt, umfasst der von der umlaufenden Dichtlippe 1a umschlossene Bereicht 1b des Maskeneinsatzes 1 eine individuelle Abformung der Nasenpartie des Patienten. Ferner sind zwei von stutzenartigen Verlängerungen umgebene Öffnungen 1e in dem Maskeneinsatz 1 vorgesehen, welche beim Tragen der Beatmungsmaske geringfügig in die Nasenlöcher des Patienten hineinragen und somit ein unmittelbares Einströmen der Atemluft in die Nase aus dem Verwirbelungsvolumen 2e des Maskenkörpers 2 ermöglichen. Hierdurch wird eine besonders effektive Beatmung des Patienten bei gleichzeitig kompakten Außenabmessungen sichergestellt.

In Figur 5 ist der flexible Maskeneinsatz 1 in einer Seitenansicht dargestellt. Gut erkennbar ist die individuell auf die Gesichtsanatomie des Patienten angepasste Dichtungslippe 1a, sowie die Ausformung des von der Dichtlippe 1a umschlossenen Bereichs 1b, welcher individuell an die Nasenpartie des Patienten angepasst ist. Zur Verdeutlichung zeigt Figur 6 einen Sagittalschnitt durch den Maskeneinsatz 1.

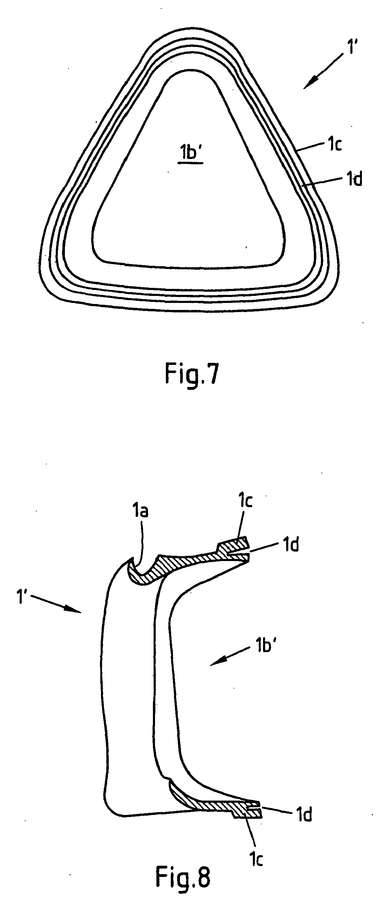

In Fig. 7 ist ein gegenüber dem Maskeneinsatz 1 der Fig. 3 konstruktiv vereinfachter Maskeneinsatz 1' dargestellt. Er unterscheidet sich von dem der Fig. 3 dadurch, dass der von der individuell an die Gesichtsanatomie des Patienten angepassten Dichtlippe 1a umschlossene Bereich 1b' durch eine im Wesentlichen dreieckige Aussparung gebildet wird, so dass bei dem Maskeneinsatz 1' die Abformung der Nasenpartie entfällt. Dies stellt gerade für solche Patienten, welche auf eine möglichst kleine Berührungsfläche zwischen dem Material des Maskeneinsatzes 1', vorliegend wiederum einem biokompatiblen medical Grade 2-Silikon-Elastomer, und der Gesichtshaut Wert legen, eine optimale Lösung hinsichtlich des Tragekomforts dar. Die absolute Dichtigkeit des Maskeneinsatzes 1' wird dabei durch die individuell an die Gesichtsanatomie des Patienten angepasste Dichtlippe 1a gewährleistet.

Fig. 8 zeigt den zugehörigen Sagittalschnitt durch den Maskeneinsatz 1'. Wie erkennbar, sind die Dichtlippe 1a, der Flansch 1c und die in den Flansch eingeformte Nut 1d gegenüber dem Maskeneinsatz der Fig. 3 im Wesentlichen unverändert.

Die vorliegende Beatmungsmaske hat den besonderen Vorteil, dass sie einerseits aus konfektionierten Komponenten, nämlich dem formsteifen Maskenkörper 2 mit der Stirnauflage 3 und dem Anschlussstück 2a für ein Beatmungsschlauch, besteht, welche kostengünstig in der Regel in mehreren Standardgrößen verfügbar sind, und andererseits über ein individuell an die Gesichtsanatomie des Patienten angepassten Maskeneinsatz 1 verfügt, welcher einen hohen Tragekomfort ebenso wie ein hohes Maß an Dichtigkeit sicherstellt. Aufgrund eines durch den Maskenkörper 2 definierten Verwirbelungsvolumens 2e kann die über den Beatmungsschlauch (nicht dargestellt) zugeführte Beatmungsluft zunächst verwirbelt werden, wobei sie sich erwärmt, bevor sie über die Öffnungen 1e des Maskeneinsatzes in die Nase des Patienten einströmt. Hierdurch wird die Beatmung angenehmer gestaltet und einem Austrocknen der Nasenschleimhäute wird wirksam begegnet.

## Patentansprüche

1. Beatmungsmaske für die Mund-Nasen- oder Nasenbeatmung, mit einem ein Anschlussstück (2a) für einen Beatmungsschlauch aufweisenden Maskenkörper (2) aus einem formsteifen Material und einem mit dem Maskenkörper (2) gesichtsseitig verbundenen, an das Gesicht des Patienten anlegbaren Maskeneinsatz (1, 1') aus einem flexiblen Material, wobei der Maskeneinsatz (1, 1') eine umlaufende Dichtlippe (1a) zur abdichtenden Anlage des Maskeneinsatzes (1, 1') am Gesicht des Patienten aufweist und wobei der Maskeneinsatz (1, 1') an seiner gesichtsabgewandten Seite mit dem Maskenkörper (2) über einen am Maskeneinsatz (1, 1') vorgesehenen zumindest teilweise umlaufenden Flansch (1c) und einen korrespondierenden, an dem Maskenkörper (2) vorgesehenen Flansch (2d) dicht verbunden ist,
wobei der Maskenkörper (2) als vorkonfektionierter Maskenkörper (2) ausgebildet ist und der Maskeneinsatz (1, 1') individuell an die Gesichtsanatomie des Patienten angepasst ausgebildet ist,
**dadurch gekennzeichnet, dass**
der Maskenkörper (2) ein Verwirbelungsvolumen (2e) aufweist, wobei die über einen Beatmungsschlauch zugeführte Beatmungsluft zunächst verwirbelt wird, so dass sich die Beatmungsluft erwärmt, bevor die Beatmungsluft über Öffnungen (1e) des Maskeneinsatzes (1, 1') in die Nase des Patienten einströmt.

2. Beatmungsmaske nach Anspruch 1,
**dadurch gekennzeichnet, dass**
der Maskeneinsatz (1) als Vollkörper ausgebildet ist, wobei der von der umlaufenden Dichtlippe (1a) umschlossene Bereich (1b) individuell an die Gesichtsanatomie des Patienten angepasst ausgebildet ist.

3. Beatmungsmaske nach Anspruch 2,
**dadurch gekennzeichnet, dass**
auch die umlaufende Dichtlippe (1a) des Maskeneinsatzes (1) individuell an die Gesichtsanatomie des Patienten angepasst ausgebildet

4. Beatmungsmaske nach Anspruch 2 oder 3,
**dadurch gekennzeichnet, dass**
der von der umlaufenden Dichtlippe (1a) umschlossene Bereich des Maskeneinsatzes (1) zumindest eine Abformung der Nasenpartie des Patienten umfasst, wobei zwei Öffnungen zum unmittelbaren Einströmen der Atmungsluft in die Nasenlöcher (1e) vorgesehen sind.

5. Beatmungsmaske nach Anspruch 4,
**dadurch gekennzeichnet, dass**
die zwei Öffnungen (1e) von stutzenartigen Verlängerungen umgeben sind, die in die Nasenlöcher des Patienten einführbar sind.

6. Beatmungsmaske nach einem der Ansprüche 1 bis 5,
**dadurch gekennzeichnet, dass**
die Beatmungsmaske einen Stützring zur Sicherung des Flansches (1c) des Maskeneinsatzes (1, 1') an dem korrespondierenden Flansch (2d) des Maskenkörpers (2) umfasst.

7. Beatmungsmaske nach einem der Ansprüche 1 bis 6,
**dadurch gekennzeichnet, dass**
der am Maskeneinsatz (1, 1') vorgesehene zumindest teilweise umlaufende Flansch (1c) eine Nut (1d) aufweist, in die eine an dem korrespondierenden Flansch (2d) des Maskenkörpers (2) vorgesehene Feder (2d*) eingreift.

8. Beatmungsmaske nach einem der Ansprüche 1 bis 7,
**dadurch gekennzeichnet, dass**
die Beatmungsmaske eine Stirnauflage (3) aufweist.

9. Beatmungsmaske nach Anspruch 8,
**dadurch gekennzeichnet, dass**
die Stirnauflage (3) arretierbar gelenkig mit dem Maskenkörper (2) verbunden ist.

10. Beatmungsmaske nach Anspruch 7 oder 8,
**dadurch gekennzeichnet, dass**
die Stirnauflage (3) wenigstens ein Stirnpolster aus einem flexiblen Material aufweist.

11. Beatmungsmaske nach einem der Ansprüche 8 bis 10,
**dadurch gekennzeichnet, dass**
der Abstand der Stirnauflage (3) von dem Maskeneinsatz (1, 1') auf Basis gesichtsanatomischer Vorgaben gewählt ist.

12. Beatmungsmaske nach einem der Ansprüche 1 bis 11,
**dadurch gekennzeichnet, dass**
das flexible Material ein biokompatibles medical Grade 2-Silikon-Elastomer ist.

## Claims

1. A respiratory mask for nose-mouth respiration or nose respiration, with a mask body (2) having a connection piece (2a) for a breathing tube and made of a dimensionally stable material, and with a mask insert (1, 1') made of a flexible material, which mask insert can be applied to the patient's face and is connected to the mask body (2) on the face side wherein the mask insert (1, 1') has a peripheral sealing lip (1a) for sealing contact of the mask insert (1, 1') with the patient's face,and wherein on its side turned away from the face, the mask insert (1, 1') is tightly connected to the mask body (2) via a flange (1c), which is provided on the mask insert (1, 1') and is at least partially peripheral, and a corresponding flange (2d), which is provided on the mask body (2),
wherein the mask body (2) is designed as a prefabricated mask body (2), and the mask insert (1, 1') is designed to be adapted individually to the patient's facial anatomy,
**characterized in that**
the mask body (2) has a turbulence volume (2e), wherein the respiratory air supplied through a breathing tube is initially swirled, so that the respiration air is heated before the respiration air flows into the patient's nose through openings (1e) in the mask insert (1, 1').

2. The respiratory mask according to Claim 1,
**characterized in that**
the mask insert (1) is designed as a solid body, wherein the area (1b) enclosed by the peripheral sealing lip (1a) is designed to be adapted individually to the facial anatomy of the patient.

3. The respiratory mask according to Claim 2,
**characterized in that**
also the peripheral sealing lip (1a) of the mask insert (1) is designed to be adapted individually to the facial anatomy of the patient.

4. The respiratory mask according to Claim 2 or 3,
**characterized in that**
the area of the mask insert (1) which is enclosed by the peripheral sealing lip (1a) comprises at least a mold of the patient's nasal area, wherein two openings are provided for direct flow of respiratory air into the nostrils (1e).

5. The respiratory mask according to Claim 4,
**characterized in that**
the two openings (1e) are surrounded by pipe-like extensions which can be inserted into the patient's nostrils.

6. The respiratory mask according to any one of Claims 1 to 5,
**characterized in that**
the respiratory mask comprises a supporting ring for securing the flange (1c) of the mask insert (1, 1') on the corresponding flange (2d) of the mask body (2).

7. The respiratory mask according to any one of Claims 1 to 6,
**characterized in that**
the at least partially peripheral flange (1c) which is provided on the mask insert (1, 1') has a groove (1d) with which a spring (2d*) that is provided on the corresponding flange (2d) of the mask body (2) engages.

8. The respiratory mask according to any one of Claims 1 to 7,
**characterized in that**
the respiratory mask has a forehead rest (3).

9. The respiratory mask according to Claim 8,
**characterized in that**
the forehead rest (3) is connected to the mask body (2) with an articulation that is lockable.

10. The respiratory mask according to Claim 7 or 8,
**characterized in that**
the forehead support (3) has at least one forehead cushion made of a flexible material.

11. The respiratory masking according to any one of Claims 8 to 10,
**characterized in that**
the distance from the forehead support (3) to the mask insert (1, 1') is selected on the basis of facial anatomy.

12. The respiratory mask according to any one of Claims 1 to 11,
**characterized in that**
the flexible material is a biocompatible medical grade-2 silicone elastomer.

## Revendications

1. Masque respiratoire pour la respiration naso-buccale ou nasale, comprenant un corps de masque (2) présentant une pièce de raccordement (2a) pour un tube respiratoire, dans un matériau indéformable, et un empiècement de masque (1, 1') relié côté visage au corps de masque (2), pouvant être posé sur le visage du patient, dans un matériau flexible, sachant que l'empiècement de masque (1, 1') présente une lèvre d'étanchéité périphérique (1a) pour poser de manière étanche l'empiècement de masque (1, 1') sur le visage du patient, et sachant que l'empiècement de masque (1, 1') est, sur son côté détourné du visage, relié de manière étanche au corps de masque (2) via une bride (1c) au moins partiellement périphérique prévue sur l'empiècement de masque (1, 1') et une bride (2d) correspondante prévue sur le corps de masque (2),
sachant que le corps de masque (2) est formé en tant que corps de masque (2) pré-confectionné et l'empiècement de masque (1, 1') est formé en étant adapté individuellement à l'anatomie du visage du patient,
**caractérisé en ce que** le corps de masque (2) présente un volume de tourbillonnement (2e), sachant que l'air respiratoire amené via un tube respiratoire est d'abord tourbillonné de façon à ce que l'air respiratoire se chauffe, avant que l'air respiratoire n'afflue dans le nez du patient via des ouvertures (1e) de l'empiècement de masque (1, 1').

2. Masque respiratoire selon la revendication 1, **caractérisé en ce que** l'empiècement de masque (1) est conçu en tant que corps intégral, sachant que la partie (1b) entourée par la lèvre d'étanchéité (1a) est formée en étant adaptée individuellement à l'anatomie du visage du patient.

3. Masque respiratoire selon la revendication 2, **caractérisé en ce que** la lèvre d'étanchéité (1a) périphérique de l'empiècement de masque (1) est également formée en étant adaptée individuellement à l'anatomie du visage du patient.

4. Masque respiratoire selon la revendication 2 ou 3, **caractérisé en ce que** la partie de l'empiècement de masque (1) entourée par la lèvre d'étanchéité (1a) comprend au moins un moulage de la partie nasale du patient, sachant que deux ouvertures sont prévues pour l'entrée directe de l'air respiratoire dans les narines (1e).

5. Masque respiratoire selon la revendication 4, **caractérisé en ce que** les deux ouvertures (1e) sont entourées par des prolongements tubulaires qui peuvent être introduits dans les narines du patient.

6. Masque respiratoire selon l'une des revendications 1 à 5, **caractérisé en ce que** le masque respiratoire comprend une bague d'appui pour fixer la bride (1c) de l'empiècement de masque (1, 1') sur la bride correspondante (2d) du corps de masque (2).

7. Masque respiratoire selon l'une des revendications 1 à 6, **caractérisé en ce que** la bride (1c) au moins partiellement périphérique prévue sur l'empiècement de masque (1, 1') présente une rainure (1d) dans laquelle se met en prise un ressort (2d*) prévu sur la bride (2d) correspondante du corps de masque (2).

8. Masque respiratoire selon l'une des revendications 1 à 7, **caractérisé en ce que** le masque respiratoire présente un appui frontal (3).

9. Masque respiratoire selon la revendication 8, **caractérisé en ce que** l'appui frontal (3) est relié au corps de masque (2) de manière articulée en pouvant être bloqué.

10. Masque respiratoire selon la revendication 7 ou 8, **caractérisé en ce que** l'appui frontal (3) présente au moins un rembourrage frontal en un matériau flexible.

11. Masque respiratoire selon l'une des revendications 8 à 10, **caractérisé en ce que** l'écart de l'appui frontal (3) à l'empiècement de masque (1, 1') est choisi en se basant sur des données d'anatomie du visage.

12. Masque respiratoire selon l'une des revendications 1 à 11, **caractérisé en ce que** le matériau flexible est un élastomère de silicone médical de degré 2 biocompatible.
